Europäisches Patentamt

European Patent Office

Office européen des brevets

(11) Publication number: 0 252 730 B1

# EUROPEAN PATENT SPECIFICATION

(45) Date of publication of patent specification: 16.09.92  (51) Int. Cl.5: C12N 9/28, C12P 19/14

(21) Application number: 87306030.5

(22) Date of filing: 08.07.87

(54) **Alpha-Amylase mixtures for starch liquefaction.**

(30) Priority: 09.07.86 US 883566

(43) Date of publication of application:
13.01.88 Bulletin 88/02

(45) Publication of the grant of the patent:
16.09.92 Bulletin 92/38

(84) Designated Contracting States:
AT BE CH DE ES FR GB GR IT LI LU NL SE

(56) References cited:
FR-A- 1 024 371
US-A- 4 284 722

PATENT ABSTRACTS OF JAPAN, vol. 11, no.
353 (C-457)[2800], 9th June 1987, page 147 C
457; & JP-A-62 126 994 (AGENCY OF IND.
SCIENCE & TECHNOL.)09-06-1987

(73) Proprietor: NOVO NORDISK A/S
Novo Allé
DK-2880 Bagsvaerd(DK)

(72) Inventor: Carrol, John O.
298 Sunrise Hill Lane
Norwalk, CT 06851(US)
Inventor: Swanson, Timothy R.
23 Whisconier Village
Brookfield Center, CT 06805(US)
Inventor: Trackman, Philip C.
Edmond Road
Bethel, CT 06801(US)

(74) Representative: Brown, John David et al
FORRESTER & BOEHMERT Franz-
Joseph-Strasse 38
W-8000 München 40(DE)

Rank Xerox (UK) Business Services

**Description**

This invention relates to starch hydrolysing enzymes. More specifically, the present invention is directed to novel α-amylase compositions and their use for the overall enzymatic conversion of starch into sugars, in particular High Fructose Corn Syrup (HFCS).

BACKGROUND OF THE INVENTION

HFCS is manufactured from high DX syrups, the term DX meaning percentage by weight of dextrose (D-glucose) calculated on the basis of dry substance (DS) of syrup. The overall enzymatic process generally adopted for conversion of starch into high DX syrup is a two-stage process. The first step is the liquefaction, i.e. the hydrolysis of starch into a mixture of oligosaccharides, the so-called maltodextrins. This process is catalyzed by α-amylases at a temperature of at least 75°C, preferably at about 90°C or by a jet-cooking process wherein the starch slurry is heated for at least several minutes to 105 - 110°C, usually with a single dose of α-amylase, and then held at about 90°C for at least 1 hour. In the primary stage of the overall liquefaction gelatinization and mechanical thinning of the starch slurry is effected. Further degradation (dextrinization) occurs in the secondary stage of the process. With respect to the jet-cooking process, reference is made to U.S. Patent No. 3,912,590.

A variety of microbial, particularly bacterial, α-amylases are commercially available for the liquefaction process, for example BAN™ (from Bacillus amyloliquefaciens) and TERMAMYL® (from Bacillus licheniformis) are supplied by NOVO INDUSTRI A/S, Denmark. α-amylases from Bacillus stearothermophilus are disclosed in U.S. patents Nos. 2,695,683 and 4,284,722. A Bacillus stearothermophilus α-amylase (THERMOLASE™) is available from Enzyme Development Corporation, NY, USA.

While BAN α-amylase is only stable up to about 85°C and hence barely suitable for the jet-cooking process, both TERMAMYL and Bacillus stearothermophilus α-amylases are well adapted for this almost globally preferred mode of starch liquefaction because they are heat stable. The subsequent saccharification step in which the maltodextrins are converted into dextrose is mostly catalyzed by a glucoamylase enzyme. Commercial glucoamylase preparations, usually derived from Aspergillus or Rhizopus species, are available from various manufacturers, e.g. as AMG™ 200 L, a product obtained from Aspergillus niger and manufactured by NOVO INDUSTRI A/S, Denmark.

The Bacillus stearothermophilus α-amylase has certain advantages over the Bacillus licheniformis enzyme, notably, a higher specific activity, a lower pH-optimum, and a modest improvement in DX of the final dextrose syrup.

The below-tabulated test results from laboratory scale comparative studies wherein the liquefaction was simulative of industrial usage circumstances illustrate the well-known fact that wereas the TERMAMYL α-amylase under jet-cooking conditions is rapidly deactivated at pH values lower than 6 even in the presence of elevated levels of $Ca^{++}$, the THERMOLASE α-amylase, on the other hand, retains a substantial activity at a dosage level equipotent to that of the TERMAMYL enzyme (vide infra) down to pH 5.5 or even lower.

However, the data also show that liquefaction with THERMOLASE entails a significant increase in the formation of sediment measured after completion of the saccharification step as compared with an equipotent dosage of TERMAMYL. Sediment formation is undesirable because, apart from causing an eventual loss of glucose it may also seriously impede the subsequent filtration of the glucose syrup.

Apparently, the problem with undue formation of sediment associated with liquefaction with THERMOLASE when used in the normal dosage range for α-amylase can be avoided by doubling the THERMOLASE dose. However, such excessive dosage levels of α-amylase are undesirable for economic reasons, the normal dosage level being in the range of 40-80 NU/g DS.

The activity standard NU (which is an abbreviation of NOVO α-amylase unit) is the amount of enzyme which hydrolyses 5.26 mg of dissolved starch per hour at 37°C, pH 5.6 and 0.0043 M of $Ca^{++}$ over a 7-20 minute reaction time.

| Liquefaction Enzyme | Sediment Volume after Saccharification (% vol/vol) Liquefaction pH | | | |
|---|---|---|---|---|
| | 5.0 | 5.5 | 5.8 | 6.0 |
| TERMAMYL® 85 NU/g DS | | 2.5*,** | 2.0* | 1.5 |
| THERMOLASE™ 50 NU/g DS | 50 | 10 | 7 | 25 |
| THERMOLASE™ 50 NU/g DS | | | 5 | |
| THERMOLASE™ 100 NU/g DS | | | 2 | |

\* Accompanied by poor liueqfaction results
\*\* At pH 5.5 twice the usual $Ca^{++}$ level was employed to stabilize the TERMAMYL®

It is an object of the present invention to devise an α-amylase preparation which under jet-cooking conditions retains its activity in the pH range of 5 to 6 without causing undue formation of sediment. According to this invention this object can be met by conducting the liquefaction process with suitable mixtures of B. licheniformis and B. stearothermophilus α-amylases.

In addition, the minor, but significant, increase in DX of the final syrup observed by using the amylase mixtures of this invention as compared with DX percentages obtained with the constituent enzymes per se, is indicative of a surprising synergistic effect obtained by combining the two amylases.

SUMMARY OF THE INVENTION

According to its first aspect the present invention provides an α-amylase composition comprising a mixture of α-amylases derived from Bacillus licheniformis and Bacillus stearothermophilus, the α-amylase mixture containing from 10 to 90% by activity measured in NU (vide infra) of Bacillus licheniformis α-amylase, based on a total α-amylase activity of the mixture of 100% measured in NU.

In a preferred embodiment of this invention the α-amylase mixture contains from 25 to 50 % by activity measured in NU of Bacillus licheniformis α-amylase.

According to a further aspect of the present invention there is provided a process for liquefying a slurry of starch or starchy grains by conducting the liquefaction process with an α-amylase composition comprising a mixture of α-amylases derived from Bacillus licheniformis and Bacillus stearothermophilus, the α-amylase mixture containing from 10-90%, preferably from 25-50%, by activity measured in NU of Bacillus licheniformis α-amylase, based on a total α-amylase activity of the mixture of 100% measured in NU.

In one preferred mode of conducting the liquefaction process, the dosage level of the α-amylase composition does not exceed 100 NU/g DS of the starch slurry.

According to a more preferred embodiment of the invention, liquefaction is conducted with a dose of the α-amylase composition in the range of from 40 to 80 NU/g DS.

According to a still more preferred embodiment the liquefaction process is conducted by jet-cooking at a temperature in the range of 100°C-115°C for 1-60 minutes, followed by reducing the temperature to be held in the range of 90°C-100°C for 30-120 minutes after which the so liquefied starch is stable against

3

retrogradation, the pH being held at 5.5-6.0 throughout the process.

DETAILED DESCRIPTION OF THE INVENTION

As has already been set forth above, the Bacillus licheniformis α-amylase of the mixture may be TERMAMYL® or any Bacillus licheniformis derived α-amylase equivalent thereto. One α-amylase from Bacillus stearothermophilus is commercially available as THERMOLASE™. Another α-amylase derived from the same species and believed to be indistinguishable from THERMOLASE is produced by a microorganism identified as ATCC No. 7954 as described in U.S. Patent No. 2,695,863. In addition, an α-amylase was produced by the inventors hereof by cultivation of a Bacillus stearothermophilus strain accorded the internal designation BPS-3. With respect to its enzyme chemical properties and immunochemical characterization the BPS-3 α-amylase has proved to be identical to THERMOLASE. Samples of this α-amylase have been available upon request to NOVO INDUSTRI A/S, DENMARK for more than one year prior to the date of this application and will continue to be so.

DEXTROZYME™ was routinely used for the saccharification experiments. It is a mixture of glucoamylase and an acidophilic, heat-stable α-1,6-glucosidase (pullulanase) and is supplied by NOVO INDUSTRI A/S, DENMARK. The enzyme mixture is described in further detail in an brochure (B 320a-GB), available upon request to the manufacturer.

Assay of α-amylase activity

The activity standard NU (which is an abbreviation of NOVO α-amylase unit) is the amount of enzyme which hydrolyses 5.26 mg of dissolved starch per hour at 37°C, pH 5.6 and 0.0043 M of $Ca^{++}$ over a 7-20 minute reaction time. A folder AF9 describing the analytical method is available on request to NOVO INDUSTRI A/S, DENMARK. At the starch liquefaction operating temperature range of 90-110°C, the THERMOLASE™ has been found to be more active than the TERMAMYL® by a factor of about 1.7. The test study results tabulated above which compared TERMAMYL® and THERMOLASE™ at starch liquefaction temperatures was an equal activity level study i.e., 50 NU/g DS of THERMOLASE™ is as effective as 85 NU/g DS of TERMAMYL® at the conditions of use.

A problem which has always been inherently connected with enzymatic starch hydrolysis is the pH level at which the liquefaction should be conducted. Starch, as such, slurried for the starch liquefaction process is at pH 3.0-5.0. Most grain slurries directly liquefied (such being employed for brewing, distilling and fuel ethanol processes) have a natural pH of 5.0-6.0 but contain significant buffering capacity. The Bacillus licheniformis α-amylase is best used at a pH 6.0-6.5. When this enzyme is employed alone at a pH of below 6.0, liquefaction results deteriorate sharply. In addition, undesireable quantities of by-products, principally maltulose, are produced when this enzyme is employed at pH values higher than 6.2.

In the high fructose syrup industry, therefore, pH of the starch slurry was adjusted upward to pH 6.0-6.5 before liquefaction with the Bacillus licheniformis α-amylase, thereby inevitably increasing the salt content of the syrup and hence the expences incurred by de-salting the final syrups, e.g. by ion exchange.

The Bacillus stearothermophilus α-amylase can operate well at pH 5.5-6.0. When starch liquefaction is conducted in this pH range, maltulose formation is substantially eliminated and colour and organic acid formation are reduced. Use of this α-amylase offers other advantages, such as a modest improvement in the ultimate dextrose yield. However, as has already been pointed out, glucose syrups made from starch liquefied with 40-80 NU/g dry starch of the Bacillus stearothermophilus α-amylase possess a high level of sediment and, therefore, filter poorly.

Although we are not bound to any theory or explanation relating to sediment formation, the surprising drop therein observed by using the α-amylase composition of this invention may be due to differences in enzyme specificity and action pattern of the respective enzymes.

Starch is formed of large complex molecules (mol. wt. above 1000 kD). It is envisioned that two α-amylases preferentially attack different parts of the starch molecule, each attacking most rapidly at sites less preferred and/or attacked more slowly by the other, and thereby, each enzyme quickly releasing fragments more susceptible to immediate attack by the other enzyme. The starch molecule is known to possess both amorphous regions and regions of high crystallinity. The crystalline regions are more resistant to hydrolysis than the amorphous regions, but become relatively more accessible to attack once bonds are broken in the neighboring amorphous regions. Then as crystalline regions are disrupted, more hydrolyzable sites are exposed.

Liquefaction is essentially the endo-attack by an α-amylase on the α-1,4-bonds of the glucose polymer chains, which significantly lowers the viscosity of the gelatinized starch.

At pH 6.5 (close to the optimum for the Bacillus licheniformis enzyme) approximately 85% of the original activity remains after secondary liquefaction. At the pH optimum for the Bacillus stearothermophilus enzyme of approximately pH 5.8, the activity remaining at the end of secondary liquefaction is 95-100%.

| Enzyme Type | pH | Secondary liquefaction % residual activity |
|---|---|---|
| TERMAMYL® | 6.5 | 85 |
| 85 NU/g DS | 6.0 | 72 |
|  | 5.75 | 63 |
|  | 5.5 | 43 |
| THERMOLASE™ | 6.0 | 74 |
| 50 NU/g DS | 5.75 | 100 |
|  | 5.5 | 83 |

The high sediment levels which result from starch liquefaction with the Bacillus stearothermophilus α-amylase are not observable in the immediate product of the primary liquefaction. The sediment might be generated in the dextrin solution during the dextrinization step. Apparently, some of the starch fragments generated by liquefaction with the Bacillus stearothermophilus enzyme become an insoluble product. Analysis of the sediment indicates presence of carbohydrate and lipid moities. When the Bacillus licheniformis α-amylase is employed at a pH lower than pH 6.0 more than the usual level of sediment also appears in the syrup solution. However, the polysaccharides in the sediment generated from starch by each of the two enzymes at pH 5.5-6.0 are not the same.

Since starch liquefaction with greater than normal enzyme dosages of the Bacillus stearothermophilus α-amylase reduces formation of sediment, and liquefaction with the Bacillus licheniformis enzyme at pH 6.0-6.5 produces low sediment levels, it is believed that failure to sever enzymatically hydrolyzable linkages (or set of linkages) in the starch molecule underlies generation of the sediment.

For further understanding of this invention, the following specific examples are presented.

Example 1

Liquefaction at pH 5.8 with an α-amylase composition containing 25% TERMAMYLYL α-amylase by activity in NU

The liquefaction process was conducted with a starch slurry containing 35% DS. The starch was supplied by Staley (corn starch, lot F 29032 8521). In all experiments $CaCl_2,2H_2O$ was used to adjust the concentration of $Ca^{++}$ to 40 ppm. NaCl was added to runs with BPS-3 α-amylase to match the salt content of TERMAMYL (final conductivity of the slurry was 200 $\mu$S).

The jet-cooking conditions were 5 minutes residence time at 105°C, followed by flash-cooling to 95°C where parallel secondary liquefaction experiments were completed after 60 and 90 minutes, respectively. The resulting maltodextrins were saccharified with DEXTROZYME™ 150/50 L (lot No. AMPP) at 60°C and pH 4.3. The dose was 0.18 amyloglucosidase units and 0.062 pullulanase units, both per g of DS of maltodextrin. Sediment volume and DX of the glucose syrup were determined after saccharification for 48 hours. The results, including those for comparative experiments conducted with TERMAMYL® and BPS-3 α-amylase alone, are presented in the following table:

## Table I

| α-amylase | Dose (NU/g) | pH | Time, second. liquefact. minutes | Saccharification (48 hours) Sediment, % (vol/vol) | DX |
|---|---|---|---|---|---|
| TERMAMYL® | 75 | 5.8 | 60 | 4.5 | 95.7 |
|  |  |  | 90 | 3.5 | 95.4 |
| BPS-3 amylase | 75 | 5.8 | 60 | 11.5 | 95.7 |
|  |  |  | 90 | 11.5 | 95.7 |
| BPS-3 amylase/ | 56.25/ | 5.8 | 60 | 8 | 96.2 |
| TERMAMYL® | 18.75 |  | 90 | 8 | 96.1 |

The data of Table I show that DX values for runs with the amylase mixture are significantly higher than those obtained in experiments conducted with separate enzymes. The sediment level in the syrup made from the mixed enzyme liquefaction is significantly lower than the sediment level from the BPS-3 catalyzed liquefaction.

Example 2

Liquefaction at pH 5.8 with an α-amylase composition containing 50% TERMAMYL α-amylase by activity in NU

The liquefaction and saccharifying conditions of Example 1 were employed with a 50/50 mixture, and with each enzyme alone. The results, tabulated below, show unacceptably high levels of sediment with THERMOLASE alone. Comparison of the DX values of the final syrups again reflects the synergistic effect of the amylases in the mixture.

6

## Table II

| α-amylase | Dose (NU/g) | pH | Time, second. liquefact. minutes | Saccharification (48 hours) Sediment, % (vol/vol) | DX |
|---|---|---|---|---|---|
| TERMAMYL® | 75 | 5.8 | 60 | 5 | 95.7 |
|  |  |  | 90 | 4.5 | 95.4 |
| THERMOLASE™ | 75 | 5.8 | 60 | 15 | 96.1 |
|  |  |  | 90 | 15 | 96.1 |
| BPS-3 amylase/ | 37.5/ | 5.8 | 60 | 5 | 96.0 |
| TERMAMYL® | 37.5 |  | 90 | 4 | 95.8 |
| THERMOLASE™/ | 37.5/ | 5.8 | 60 | 4.5 | 96.2 |
| TERMAMYL® | 37.5 |  | 90 | 4.5 | 96.1 |

Example 3

Filterability of glucose syrups

Example 2 was repeated with 90 minutes holding time in the secondary liquefaction stage. The saccharification results (not included herein) were consistent with the data provided in Example 2.

The filtration rates for the syrups were ascertained by the filter test leaf method, results being reported as the average of about 20 filtration cycles in ml/cycle. The 1.0 proportion of syrup insolubles was measured as percent by weight of DS. The results are tabulated below.

## Table III

| | Sediment % (vol/vol) | Filtration rate (ml/cycle) | Insolubles % of DS |
|---|---|---|---|
| TERMAMYL® | 4 | 110 | 1.2 |
| TERMAMYL® | 5 | 94 | 1.3 |
| BPS-3 amylase | 12 | 70 | 3.4 |
| TERMAMYL®/ BPS-3 amylase | 4 | 99 | 1.5 |

The features disclosed in the foregoing description and in the following claims may, both separately and in any combination thereof, be material for realising the invention in diverse forms thereof.

**Claims**

1.  An α-amylase composition comprising a mixture of α-amylases derived from Bacillus licheniformis and Bacillus stearothermophilus, said α-amylase mixture containing from 10-90% by activity measured in NU (NOVO α-amylase unit is the amount of enzyme which hydrolyses 5.26 mg of dissolved starch per hour at 37°C, pH 5.6 and 0.0043 M of Ca$^{++}$ over a 7-20 minute reaction time) of Bacillus licheniformis α-amylase, based on a total α-amylase activity of the mixture of 100% measured in NU.

2.  The α-amylase composition according to Claim 1, wherein the α-amylase mixture contains from 25-50% by activity measured in NU of Bacillus licheniformis α-amylase.

3.  A process for liquefying a slurry of starch or starchy grains, characterized by conducting the liquefaction process with an α-amylase composition comprising a mixture of α-amylases derived from Bacillus licheniformis and Bacillus stearothermophilus, said α-amylase mixture containing from 10-90% by activity measured in NU (NOVO α-amylase unit is the amount of enzyme which hydrolyses 5.26 mg of dissolved starch per hour at 37°C, pH 5.6 and 0.0043 M of Ca$^{++}$ over a 7-20 minute reaction time) of Bacillus licheniformis α-amylase, based on a total α-amylase activity of the mixture of 100% measured in NU.

4.  The process of Claim 3, wherein the α-amylase mixture contains from 25-50% by activity measured in NU of Bacillus licheniformis α-amylase, based on a total α-amylase activity of the mixture of 100% measured in NU.

5.  The process of Claim 3-4, wherein the dosage level of the α-amylase composition does not exceed 100 NU per g of dry solids (DS) of the starch slurry.

6.  The process of Claim 5, wherein the dose of α-amylase composition is in range of from 40-80 NU per g DS.

7.  The process of Claim 3-6, wherein liquefaction is conducted by jet-cooking at a temperature in the range of from 100-115°C for 1-60 minutes, followed by reducing the temperature to 90-100°C and holding the reaction mixture for 30-120 minutes after which the so liquefied starch is stable against retrogradation, the pH being held at 5.5-6.0 throughout the process.

**Patentansprüche**

1.  α-Amylase-Zusammensetzung, welche eine Mischung aus von Bacillus licheniformis und Bacillus stearothermophilus abstammenden α-Amylasen umfaßt, wobei besagte α-Amylase-Mischung von 10-90 % nach Aktivität, gemessen in NU (NOVO-α-Amylase-Einheit ist die Menge an Enzym, welche 5,26 mg aufgelöste Stärke pro Stunde bei 37°C, pH 5,6 und 0,0043 M Ca$^{++}$ über eine Reaktionszeit von 7-20 Minuten hydrolysiert), von α-Amylase aus Bacillus licheniformis enthält, bezogen auf eine Gesamt-α-Amylase-Aktivität der Mischung von 100 %, gemessen in NU.

2.  α-Amylase-Zusammensetzung nach Anspruch 1, dadurch gekennzeichnet, daß die α-Amylase-Mischung von 25-30 % nach Aktivität, gemessen in NU, von α-Amylase aus Bacillus licheniformis enthält.

3.  Verfahren zur Verflüssigung einer Aufschlämmung von Stärke oder stärkehaltigen Körnern, dadurch gekennzeichnet, daß das Verflüssigungsverfahren mit einer α-Amylase-Zusammensetzung durchgeführt wird, welche eine Mischung aus von Bacillus licheniformis und Bacillus stearothermophilus abstammenden α-Amylasen umfaßt, wobei besagte α-Amylase-Mischung von 10-90% nach Aktivität, gemessen in NU (NOVO-α-Amylase-Einheit ist die Menge an Enzym, welche 5,26 mg aufgelöste Stärke pro Stunde bei 37°C, pH 5,6 und 0,0043 M Ca$^{++}$ über eine Reaktionszeit von 7-20 Minuten hydrolysiert) von α-Amylase aus Bacillus licheniformis enthält, bezogen auf eine Gesamt-α-Amylase-Aktivität der Mischung von 100 %, gemessen in NU.

4.  Verfahren nach Anspruch 3, dadurch gekennzeichnet, daß die α-Amylase-Mischung von 25-50% nach

EP 0 252 730 B1

Aktivität, gemessen in NU, von α-Amylase aus Bacillus licheniformis enthält, bezogen auf eine Gesamt-α-Amylase-Aktivität der Mischung von 100 %, gemessen in NU.

5. Verfahren nach den Ansprüchen 3 bis 4, dadurch gekennzeichnet, daß das Dosierungsniveau von α-Amylase-Zusammensetzung nicht 100 NU pro g Trockenfeststoff (DS) der Stärkeaufschlämmung überschreitet.

6. Verfahren nach Anspruch 5, dadurch gekennzeichnet, daß die Dosis der α-Amylase-Zusammensetzung in einem Bereich von 40 bis 80 NU pro g DS ist.

7. Verfahren nach den Ansprüchen 3 bis 6, dadurch gekennzeichnet, daß Verflüssigung durchgeführt wird durch Düsenkochen bei einer Temperatur im Bereich von 100-115°C für 1-60 Minuten, gefolgt von Reduktion der Temperatur auf 90-100°C und Halten der Reaktionsmischung für 30-120 Minuten, nach welchen die so verflüssigte Stärke stabil gegenüber Retrogradation ist, wobei der pH während des Verfahrens auf 5,5-6 gehalten wird.

**Revendications**

1. Composition d'α-amylase comprenant un mélange d'α-amylases dérivées du Bacillus licheniformis et du Bacillus stéarothermophilus, le mélange d'α-amylase contenant de 10-90 % par activité mesurée en NU (une unité α-amylase NOVO est la quantité d'enzyme qui hydrolyse 5,26 mg d'amidon dissous par heure à 37°C, pH 5,6 et 0,0043 M de Ca$^{++}$ pendant une durée réactionnelle de 7-20 minutes) d'α-amylase Bacillus licheniformis, basée sur un total d'activité α-amylase du mélange de 100 % mesurés en NU.

2. Composition d'α-amylase selon la revendication 1, dans laquelle le mélange α-amylase contient de 25-50 % par activité mesurés en NU d'α-amylase de Bacillus licheniformis.

3. Procédé destiné à la liquéfaction d'une bouillie d'amidon ou de grains d'amidon, caractérisé par l'étape consistant à conduire l'opération de liquéfaction avec une composition d'α-amylase comprenant un mélange d'α-amylases dérivées du Bacillus licheniformis et du Bacillus stéarothermophilus, le mélange d'α-amylase contenant de 10-90 % par activité mesurés en NU (une unité α-amylase NOVO est la quantité d'enzyme qui hydrolyse 5,26 mg d'amidon dissous par heure à 37°C, pH 5,6 et 0,0043 M de Ca$^{++}$ pendant une durée réactionnelle de 7-20 minutes) d'α-amylase Bacillus licheniformis, basée sur un total d'activité α-amylase du mélange de 100 % mesurés en NU.

4. Procédé selon la revendication 3, dans lequel le mélange d'α-amylase contient de 25-50 % par activité mesurés en NU d'α-amylase de Bacillus licheniformis basée sur une activité d'α-amylase totale du mélange de 100 % mesurés en NU.

5. Procédé selon la revendication 3-4, dans lequel le taux de dosage de la composition d'α-amylase ne dépasse pas 100 NU par gramme de matières sèches (DS) de la bouillie d'amidon.

6. Procédé selon la revendication 5, dans lequel la dose de la composition d'α-amylase se situe dans la plage allant de 40-80 NU par gramme de matières sèches (DS).

7. Procédé selon la revendication 3-6, dans lequel la liquéfaction est conduite par cuisson au jet à une température dans la plage allant de 100-115°C pendant 1-60 minutes, suivie de la réduction de la température jusqu'à 90-100°C et maintien du mélange réactionnel pendant 30-120 minutes après quoi l'amidon ainsi liquéfié est stable contre la rétrogradation, le pH étant maintenu à 5,5-6,0 pendant tout le procédé.

9